Europäisches Patentamt

(19) European Patent Office

Office européen des brevets

(11) Publication number: **0 200 174**
**B1**

(12) **EUROPEAN PATENT SPECIFICATION**

(45) Date of publication of patent specification: **02.11.89**

(51) Int. Cl.⁴: **A 61 G 7/02, A 61 G 9/00**

(21) Application number: **86105748.7**

(22) Date of filing: **25.04.86**

(54) **Device for automatic rinsing of private parts after defecation and/or urination of physically disabled persons.**

(30) Priority: **27.04.85 JP 91743/85**
**11.06.85 JP 125138/85**

(43) Date of publication of application:
**05.11.86 Bulletin 86/45**

(45) Publication of the grant of the patent:
**02.11.89 Bulletin 89/44**

(84) Designated Contracting States:
**AT BE CH DE FR GB IT LI NL SE**

(56) References cited:
**GB-A-2 050 838**
**US-A-3 626 941**
**US-A-3 757 355**

(73) Proprietor: **Taniguchi, Harushige**
**4-432, Ikebukuro Toshima-ku**
**Tokyo (JP)**

(72) Inventor: **Taniguchi, Harusige**
**4-432, Ikebukuro Toshima-ku**
**Tokyo (JP)**
Inventor: **Taniguchi, Kyoko**
**4-432, Ikebukuro Toshima-ku**
**Tokyo (JP)**

(74) Representative: **Strehl, Schübel-Hopf, Groening, Schulz**
**Maximilianstrasse 54 Postfach 22 14 55**
**D-8000 München 22 (DE)**

## Description

This invention relates to a device for automatic rinsing of private parts after defecation and/or urination. More particularly, it relates to a device for rinsing of private parts at the time of defecation and/or urination of physically disabled persons, including the aged and patients.

In the currently used automatic rinsing devices, sensors are provided to the toilet bowl for sensing the defecation and/or urination and the rinsing liquid is discharged for a predetermined time with the aid of a timer for rinsing the private parts of the user such as anus. However, these known devices are adapted for healthy persons who can sit on the toilet during rinsing. These devices are not effective with physically disabled persons whose bodily movements are not at their will since they cannot get their private parts rinsed sufficiently. If they should try to wash their private parts at any rate, the rinsing liquid such as warm water will be scattered occasionally without being used effectively for rinsing.

US—A—3,626,941 discloses a device for collecting urine and faeces excreted from a user's body and for rinsing the private parts of said user's body after urination and defecation, said device, in its main part, including means for temporarily securing the user's body to the device while covering private parts, means for collecting urine and faeces excreted from the user's body, inlet ports for introducing and drainage ports for discharging a rinsing liquid and means for releasing the rinsing liquid for a predetermined time towards the private parts of the user's body. However, this device has to be started by a manually activated timer to provide cleansing and drying of the private parts.

From GB—A—2 050 838, an automatic urine collecting system is known which includes an automatic rinsing system for the corresponding parts of the user's body, said system including means for sensing urination to thereby release the rinsing liquid automatically. However, discharge means for faeces are not provided by the system disclosed in said document.

In our copending patent application (Japanese Laid-open Patent Publication No. 155252/1984) entitled "Device for Automatic Disposal of Bodily Waste Materials", the user's waist in its entirety is accommodated in a hermetically sealed container and both the shower and the warm air are introduced into the container. This device also is not completely satisfactory since it tends to be bulky and in need of a large amount of rinse water while unnecessarily restricting bodily movements of the users who can rise or sit on the bed at their will.

It is a principal object of the present invention to provide a device for automatic rinsing of private parts after defecation and/or urination of physically disabled persons while lying or sitting on the bed.

It is a further object of the present invention to provide a device for automatic rinsing of private parts after defecation and/or urination of physically disabled persons, which intimately contacts portions of the user's body to prevent scattering of the rinsing liquid.

It is a further object of the present invention to provide a device for automatic rinsing of private parts for physically disabled persons, which is easy to use and simple in structure.

The above and other objects of the invention will become apparent from the following description.

In view of these objects, the present invention provides device for collecting urine and faeces excreted from a user's body and for automatically rinsing private parts of said user's body after the urination and/or defecation comprising a main body including means for temporarily securing the user's body to the device while covering private parts such as anus and urethra, an inlet port for introducing a rinsing liquid for rinsing the private parts, a drainage port for discharging the used rinsing liquid with discharge materials out of the main body; and means for releasing the rinsing liquid for a predetermined time towards the private parts such as anus and urethra, said device being characterized by said main body including means for sensing urination and defecation, means being provided for discriminating defecation and urination from each other on the basis of the output signals from said sensor means, and by said release of the rinsing liquids being carried out on the basis of the results of discrimination by said discriminating means.

Fig. 1 is a perspective view showing a device according to a first embodiment of the present invention, the device being used solely by female users for rinsing their private parts after defecation and/or urination;

Fig. 2 is a central longitudinal section through the device shown in Fig. 1;

Fig. 3 is a top plan view of Fig. 1;

Fig. 4 is an exploded perspective view of Fig. 1;

Fig. 5 is a perspective view of a modified device used solely by male users and operating on the same principle as the embodiment of Fig. 1;

Fig. 6 is a perspective view of a further modified device used solely by bedridden female users and operating on the same principle as the embodiment of Fig. 1;

Fig. 7 is a view showing the operating state of the device shown in Fig. 6;

Fig. 8 is a perspective view showing the device of Fig. 5 which is adapted to be used by bedridden male users;

Fig. 9 is a view showing the operating state of the device shown in Fig. 8.

Fig. 10 is a side elevation showing the device of Fig. 1 or Fig. 5 in conjunction with a reclining type bed.

Fig. 11 is a side elevation, shown partially in section and showing a device for automatically rinsing private parts after defecation and/or urination of the female user according to a modified embodiment of the present invention;

Fig. 12 is a perspective view of a modified device adapted to be used by bedridden female users and operating under the same operating principle as the device of Fig. 11;

Fig. 13 is a perspective view of another modified device adapted to be used by bedridden male users and operating under the same operating principle as the device of Fig. 11;

Fig. 14 is a block diagram showing a sensor unit, a defecation/urination discriminating unit and a rinsing liquid discharging unit of the device shown in Fig. 1;

Fig. 15 shows a timing chart showing the state of rinsing after defecation by the rinsing device shown in Fig. 1;

Fig. 16 shows a timing chart showing the state of rinsing after urination by the rinsing device shown in Fig. 1;

Fig. 17 shows a timing chart showing the state of rinsing after defecation and urination by the rinsing device shown in Fig. 1;

Fig. 18A(i), 18B(ii) and 18C(iii) show the step of discarding cold water, the step of rinsing and the end of rinsing by the operation of the magnetic valve for urination of the device shown in Fig. 11, respectively, wherein Figs. 18A(a), 18B(b) and 18C(c) show the step of discarding cold water, the step of rinsing and the end of rinsing by the operation of the magnetic valve for defecation, respectively, of the device shown in Fig. 11;

Fig. 19 shows a modification in which the piping for rinsing the anus and that for rinsing the urethra are branched at the water heater;

Figs. 20A and 20B show further modified forms of Fig. 19;

Fig. 21 is a block diagram showing the sensor unit, defecation/urination discriminating unit and the rinsing liquid discharging unit of the rinsing device of Fig. 11;

Fig. 22 is a timing chart showing the state of rinsing after urination by the device shown in Fig. 11;

Fig. 23 is a timing chart showing the state of rinsing after defecation by the device shown in Fig. 11; and

Fig. 24 is a timing chart showing the state of rinsing after defecation and urination by the device shown in Fig. 11.

The automatic rinsing device according to preferred embodiments of the present invention will be hereafter explained by referring to the accompanying drawings.

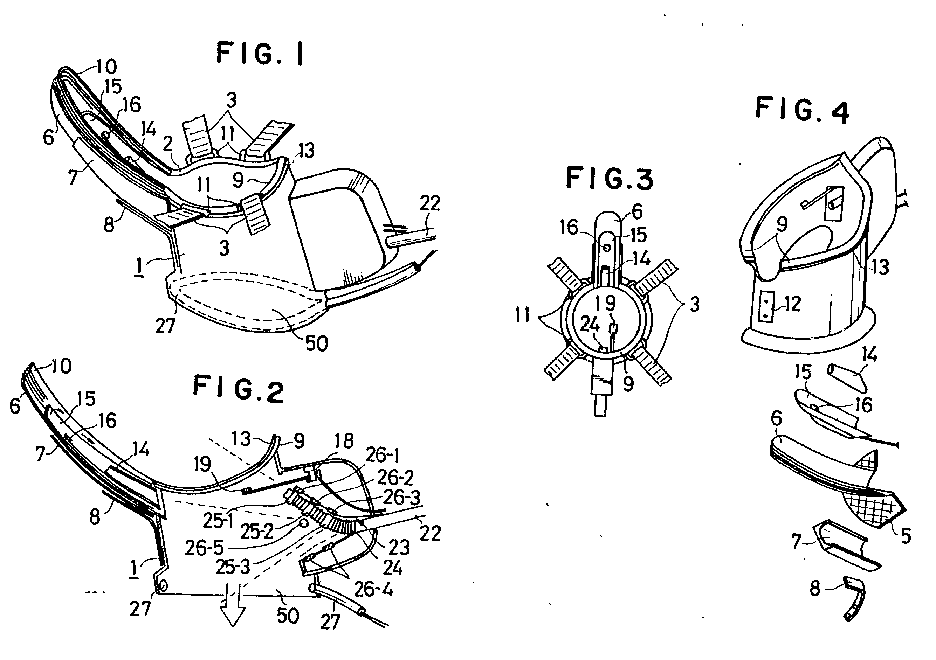

Referring first to Figs. 1 to 4, the main body 1 of the rinsing device has an upper opening water-tightly contacting with private parts including a defecation opening (anus) and a urination opening (urethra) of the user's body. It has four upper attachment members 11 for engagement with four belts 3 acting as means for temporarily attaching the device to the user's body. A safety cover 6 for inhibiting water leakage is mounted integrally to the main body 1 at a juncture 12 along with a lower base 7 and a spring 8 in such a manner that the upper opening of the main body 1 will overlie not only the anus which is the opening for defecation but also the urethra which is the opening for urination of the female user. The cover 6 is comprised of a base joint section 5 and an elongated trough 6 extending from the joint section. The spring 8 is adapted to press the water leakage safety cover 6 into intimate fitting with the private portions of the user's body. A shock-absorbing bellows-like member 10 is provided at the upper portion of the cover 6 which is the portion to contact with the user's body, in such a manner that the bellows-like member 10 is contiguous to a water-proofing member 9 provided on an upper frame 13 at the upper opening of the main body 1. The bellows-like member 10 is used as a packing to water-tightly seal the contacting portion with the user's body to prevent the rinsing liquid from leaking to outside of the rinsing device in cooperation with the water-proofing cushioning member 9. Inside the cover 6 is a reflux member or deflector 15 adapted to guide the rinsing liquid from the rinse liquid nozzle towards the urethral opening for rinsing the private portion including the urethral opening to then return the used rinsing liquid towards the toilet bowl. To this deflector 15 is attached a temperature and/or humidity sensor 16 to sense the urination. The output signals from the sensor are coupled to a defecation/urination discriminating unit which will be described. A guide piping 14 is adapted to guide the rinsing water from the nozzle towards the urethral opening and secured to the reflux member 15 by welding or with an adhesive.

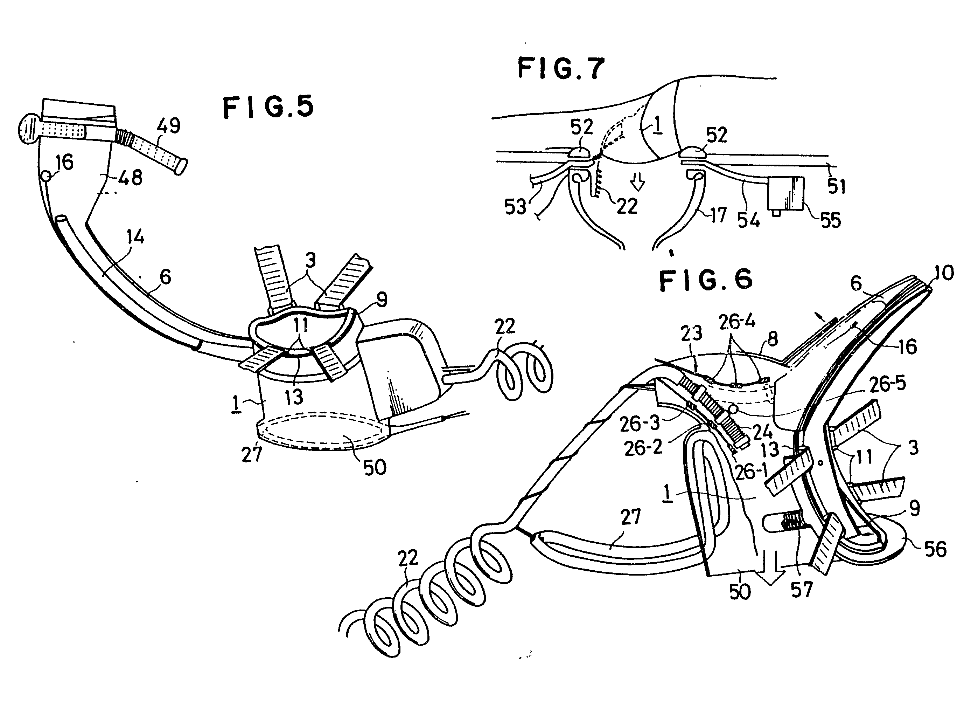

Below the main body 1 is a discharge opening 50 communicating with a toilet bowl 17, Fig. 7, for discharging the used rinsing water with the waste material. The opening 50 is connected with the toilet bowl lying in the direction of an arrow mark in Fig. 2 either directly or via a connecting hose, not shown, preferably an electric heater 27 for warming the private parts is provided on the perimeter of the opening 50, the temperature of which is controlled to a preset constant value by a thermostat, not shown. In addition to the sensor 16, a pressure sensor 19 is also provided within the main body 1 of the rinsing device, the pressure sensor being disposed below the anus which is the opening for defecation, while also being vertically movable about a fulcrum 18. The signal from the sensor 19 is supplied to a defecation/urination discriminating unit as later described.

Although the rinsing water may be supplied from the water main directly, the water supplied from the main may be stored in a water tank 20 and then supplied to a water heater 21 where it is heated to a suitable temperature and transmitted as rinsing warm water through a piping 22.

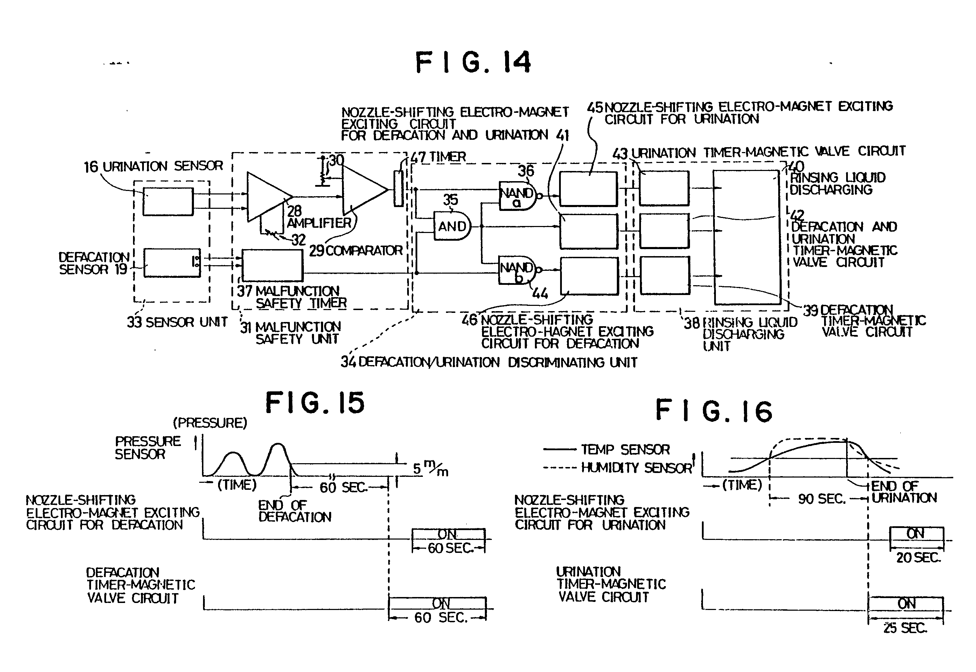

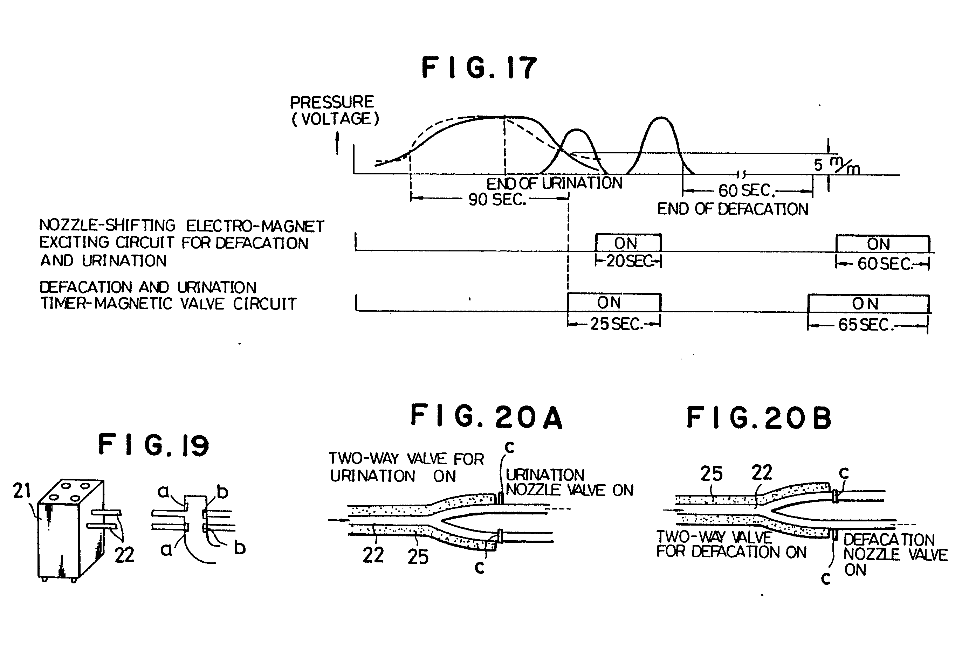

Referring to Fig. 2, a nozzle 24 is disposed in a water inlet port 23 and provided at predetermined distances with a plurality of iron segments 25-1, 25-2 and 25-3 operatively associated with timer-electro-magnets 26-1, 26-2, 26-3, 26-4 and 26-5. These electro-magnets are excited in dependence upon urination and/or defecation as sensed by the aforementioned sensors for shifting the nozzle 24 towards the opening for defecation and/or that for urination. Referring more specifically to Figs. 14 and 17, in case of urination, an output signal indicating the state of urination from changes in temperature or humidity rises sharply through amplifier 28 of a malfunction safety unit 31. The amplified signal is compared in a comparator 29 with a voltage previously set on a potentiometer 30. When the output from the comparator 29 exceeds a preset value, timer 47 is started to produce a signal to command the discharge of rinsing water to take place after lapse of a certain time, as in 90 seconds after the end of urination, as shown in Fig. 16. The numeral 32 denotes a variable resistor for calibrating the operating temperature of the temperature sensor 16 of the sensor unit 33 as a function of seasonal changes in ambient atmospheric temperature. The signal from the comparator 29 of the unit 31 is passed through AND gate 35 and NAND *a* circuit 36 of a defecation/urination discriminating unit 34 in order to recognize urination. By the operation of a nozzle-shifting electro-magnet for urination-timer-magnet exciting circuit 45, a pair of electro-magnets 26-5 on both sides of the main body 1 are excited for shifting the nozzle 24 from its normal position facing to the electro-magnet 26-4 in the direction of the guide pipe 14 for holding the nozzle in this position for 20 seconds, see Figs. 2 and 16. The signal indicating urination at the defecation/urination discriminating unit 34 causes an electro-magnet, not shown, of the water inlet port 23 to be opened with the aid of a urination timer-magnetic valve circuit 43 for emitting a rinsing liquid at 40 from the nozzle for 25 seconds in the direction of the guide pipe 14, Figs. 2 and 16. The circuit 45 is fitted with the timer in order that the nozzle will be transferred in the direction of the guide pipe 14 after lapse of 5 seconds since the opening of the electro-magnet 45 for emitting the warm rinsing liquid from the water heater to the private parts of the body, Fig. 16. In this manner, the water which will flow in cold state from the water heater to the nozzle port in winter is not discharged directly to the private portions of the user's body.

Then, as shown in Figs. 14 and 15, the signal that has sensed the state of urination at the pressure sensor 19 of the sensor unit 33 ceases for 60 seconds in order to wait for complete cessation of defecation with the aid of the timer 37 of the malfunction safety unit 31. The signal is then passed through AND gate 35 and NAND *b* gate 44 of the unit 34 to recognize the defecation to then excite the electro-magnets 26-1, 26-2 and 26-3 with the aid of a nozzle-shifting electro-magnet-timer-magnet exciting circuit 46 for shifting the nozzle 24 in the direction of the anus which is the opening for defecation so that the nozzle 24 will remain in this position for about 60 seconds, see Fig. 2. The signal indicative of the state of defecation in the defecation/urination discriminating unit 34 causes the magnetic valve, not shown, of the water inlet port 23 to be opened with the aid of a defecation timer-magnetic valve circuit 39 of a rinsing liquid discharging unit 38 for discharging the rinsing liquid at 40 for 65 seconds from the nozzle to the anus which is the opening of defecation. The nozzle-shifting electro-magnet for defecation-timer-magnet exciting circuit can be designed, as shown in Fig. 15, to shift the nozzle in 5 seconds after the opening of the magnetic valve to prevent the cold water from being discharged to the private portions.

Then, referring to Figs. 14 and 17, the signals indicative of both the defecation and urination at the pressure sensor 19 and the temperature or humidity sensor 16 of the sensor unit 33 are passed through the malfunction safety unit 31 to recognize both the defecation and urination at the AND gate 35 of the discrimination unit 34. The resulting signal is transmitted via nozzle-shifting electro-magnet-timer-magnet exciting circuit 41 for sequentially exciting electro-magnets 26-5, 26-1, 26-2 and 26-3 with the aid of the timer in such a manner that, in 90 seconds after the urination signal has first reached a predetermined voltage value, the nozzle is directed towards the opening for urination and a rinsing liquid is discharged for 25 seconds for rinsing the opening for urination for 20 seconds, and that, in 60 seconds after the end of defecation, the nozzle is directed towards the anus and the magnetic valve, not shown, is opened by the defecation/urination timer-magnetic valve circuit 42 of the rinsing liquid discharging unit 38 to discharge the rinsing liquid for 65 seconds. It will be noted that the rinsing liquid is to be discharged for 5 seconds before nozzle shifting so as not to cause cold water to be discharged to the private portions of the user's body.

Fig. 5 shows the inventive automatic rinsing device adapted for male users. The device shown in Fig. 5 differs from the embodiment shown in Figs. 1 through 4 in that the leakage safety cover 6 is a long tube in which a temperature or humidity sensor 16 for sensing the urination and a guide pipe 14 are led closely to a washing pouch 48 into which the opening of male user's urethral opening is introduced and that a fastener 49 including a hook, loop and a string for attachment to a penis is mounted to the washing pouch 48. In other respects, the present embodiment is similar to the device for the female user described in connection with Figs. 1 through 4.

Fig. 6 shows an automatic rinsing device which is used by female users as the embodiment shown in Fig. 1. The present embodiment differs from the device shown in Fig. 1 in that it is adapted to be used by bedridden patients. The rinsing device of the present device has a water inlet port 23 from the water supply system at the front lower side of the main body 1 and a water discharge port 50 communicating with toilet bowl 17 (Fig. 7) at the rear side of the main body 1. In addition, the timer-nozzle-shifting electro-magnets 26-1, 26-2, 26-3, 26-4 and 26-5 of the defecation/urination discriminating unit 34 and the nozzle 24 are provided to the water inlet port 23. The nozzle-shifting electro-magnet for urination-

timer-magnet exciting circuit 45 is operatively associated with the timer-nozzle-shifting electro-magnet for urination 26-4. Similarly, the nozzle-shifting electro-magnet for defecation-timer-magnet exciting circuit 46 is operatively associated with the timer-nozzle-shifting electro-magnet for defecation 26-5, and the nozzle-shifting electro-magnet for defecation/urination-timer-magnet exciting circuit 41 is operatively associated with the timer-nozzle-shifting electro-magnets for defecation/urination 26-4 and 26-5, with the timer-nozzle-shifting electro-magnets 26-1, 26-2 and 26-3 indicating the normal position for the nozzle 24. In Fig. 6, the numeral 56 denotes a water deflector below the user's buttock and the numeral 57 a spring adapted to fit the water-cushioning member 9 with the buttock. Fig. 7 shows the state in which the present rinsing device is mounted in position to permit the user to urinate on a seat 52 of the toilet bowl mounted on the bed. In the drawing, the numeral 53 denotes a front side warm air discharge port from an air heater, not shown, and the numeral 54 a rear side warm air discharge port from an air heater 55 adapted for drying the private parts after rinsing.

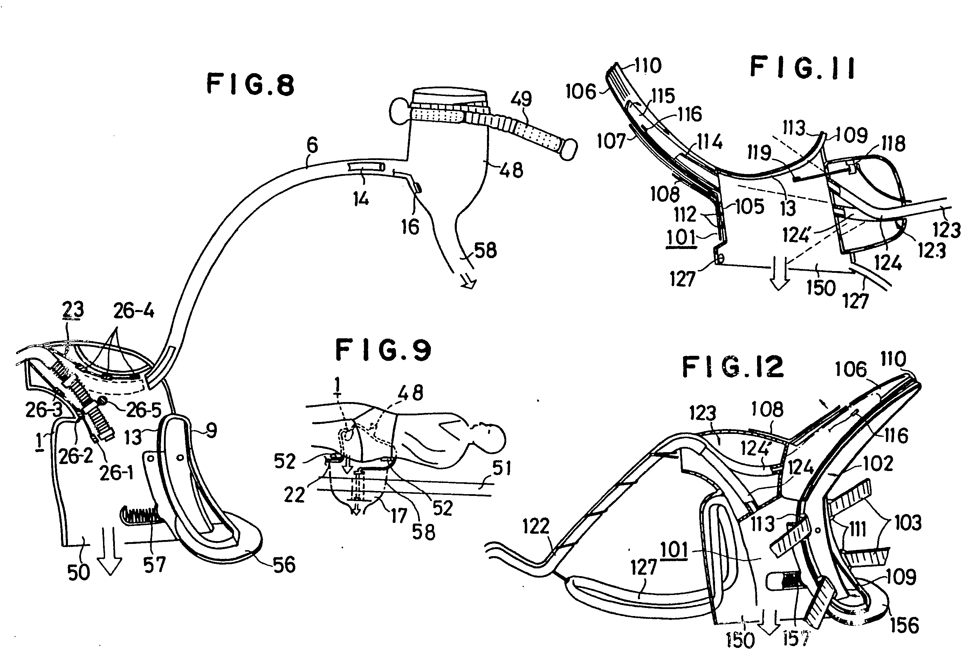

Fig. 8 shows an automatic rinsing device for males similar to the embodiment shown in Fig. 5. The device of Fig. 8 differs from the device shown in Fig. 5 in that it is used by bedridden users. The device shown in Fig. 8 has a water inlet port 23 from the water supply device at the front lower side of the main body 1 and a water discharge port 50 communicating with the toilet bowl 17 at the rear side of the main body 1. The device shown in Fig. 8 also has a nozzle 24, timer-nozzle-shifting electro-magnets 26-1, 26-2, 26-3, 26-4 and 26-5, and a drain tube 58 in the pouch 48 for draining the used rinse water and urine into the toilet bowl 17. The parts used in the device of Fig. 8 are configured similarly to the corresponding parts used in Fig. 6. Fig. 9 shows the state in which the device shown in Fig. 8 is mounted in position to permit the user to defecate on the seat of the toilet bowl 17 mounted on the bed.

Fig. 10 shows the state in which the device shown in Fig. 1 or 5 is used in conjunction with a reclining type bed 51 to permit the user to defecate with a part of the bed used as a backrest. In this figure, the numeral 61 designates a tank in which is stored the waste material delivered from the toilet bowl 17 through a waste material drainage pipe 62 and a vacuum unit, not shown. The reclining can be achieved by extending or contracting a pair of cylinders 59 and 60 by the operation of a driving circuit, not shown, on the basis of the output signals from the sensor 33 at the time of urination and/or defecation, Figs. 1 and 5.

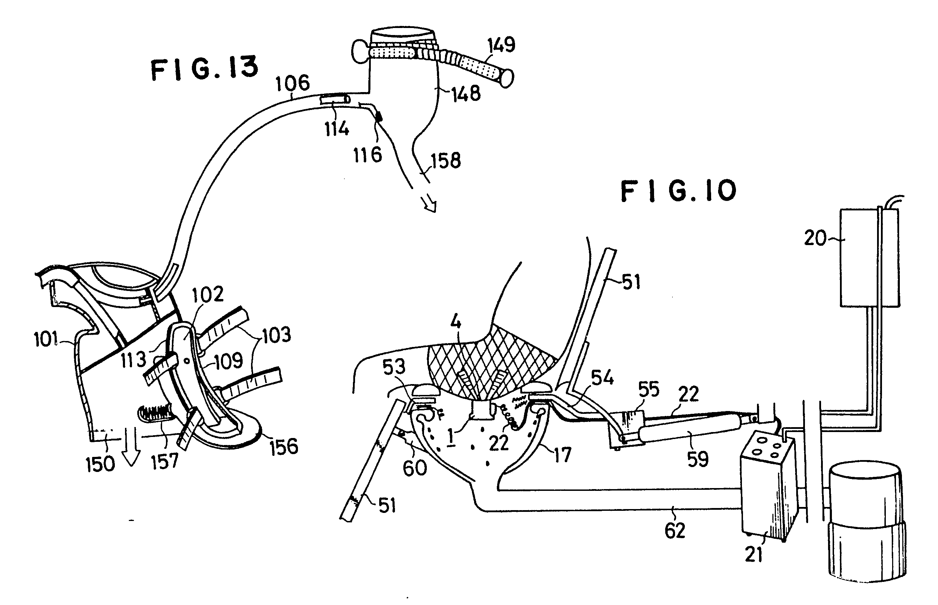

The automatic rinsing device according to a modified embodiment of the present invention will be explained by referring to Figs. 11 through 13. The device shown in Fig. 11, corresponds to the device shown in Figs. 1 to 5. The device shown in Fig. 12 corresponds to the device shown in Fig. 6, while the device shown in Fig. 13 corresponds to the device shown in Fig. 8. The devices shown in Figs. 11, 12, and 13 are similar to the devices shown in Figs. 1—5, 6 and 8 of the first embodiment except that stationary nozzles 124, 124' are used in place of the movable nozzle 24 provided with the iron segments 25-1, 25-2 and 25-3 and the timer-electro-magnets 26-1, 26-2, 26-3, 26-4 and 26-5. For this reason, the parts shown in Figs. 11 through 13 and similar to those used in the above described first embodiment are denoted by the same numerals but added by 100 and the corresponding description is omitted. Similarly, when reference is made to Figs. 18 through 24 for explaining the second embodiment, the same or similar parts as those used in the first embodiment are denoted by the same reference numerals added by 100 and the corresponding description is not made for simplicity.

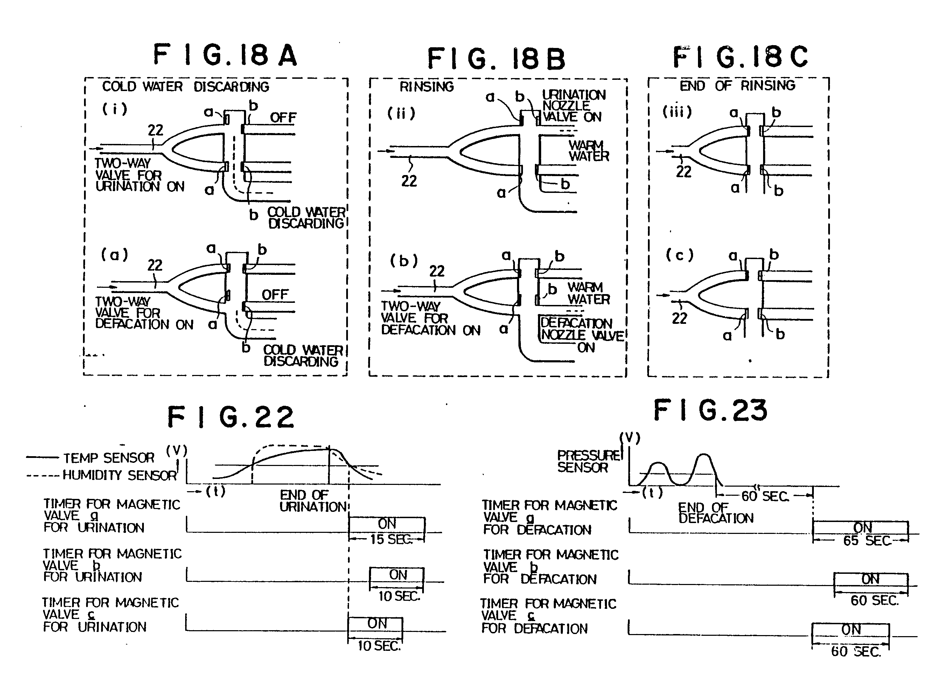

Referring now to Figs. 18 and 21, the signal from a comparator 129 of a malfunction safety unit 131 is transmitted through an AND gate 135 and a NAND a gate 136 of the defecation/urination discriminating unit 134 to recognize only urination. By signals from the unit 134 indicating urination, the magnetic valves a and b for urination between the water supply device and the water supply port are opened with the aid of the urination timer-magnetic valve circuit 143 of the rinsing liquid discharging unit 138 to discharge rinsing liquid at 140 for 10 seconds from the nozzle towards the guide pipe 114. Referring more particularly to Figs. 18A(i), 18B(ii), 18C(iii) and 22, the 15-second timer of the magnetic valve a for urination is actuated to turn on the magnetic valve a for urination to discard the cold rinsing water from the water heater 121 (Fig. 19) to the valve into the toilet bowl 17, see (i). The magnetic valve b for urination is turned on for 10 seconds by the 10-second timer of the magnetic valve b for urination for emitting the rinsing liquid to the private parts, see (ii), the valves a and b being then turned off, see (iii). In this manner, there may be avoided a situation in which the cold water between the valve and the water heater is emitted directly to the private parts during the aforementioned 5 seconds in winter.

In Figs. 11, 18A, B and C, the water passage between the water heater 121 and the valve is divided into a defecation nozzle 124 and a urination nozzle 124' with the use of a bifurcated pipe. Alternatively, the piping for defecation and that for urination may be directly divided from the water heater 121. Fig. 20 shows a further embodiment in which a heat reserving device 125 such as a heater is provided in the water passage between the water heater 121 and the valve. In this case, the timer for the magnetic valve c for urination may be turned on for 10 seconds by the operation of the circuit 145 to turn on the magnetic valve c for urination, Fig. 20A, so that the warm water will be discharged at 140 to the private parts of the user's body.

The signal from the pressure sensor 119 of the sensor unit 133 indicating defecation, Fig. 21, is transmitted to the malfunction safety circuit 137 of the malfunction safety unit 131 where the signal ceases for 60 seconds in order to wait for complete cessation of defecation. The output signal from the unit 131 is then transmitted through the AND gate 135 and NAND *b* gate 144 of the defecation/urination discriminating unit 134 in order to ascertain excretion of faeces. On the other hand, the signal from the unit 134 indicating defecation causes the magnetic valves *a* and *b* between the water supply unit and the water inlet port 123 to be opened with the aid of the defecation timer-magnetic valve circuit 139 of the rinse liquid discharging unit 138 to discharge the rinsing liquid at 140 for 60 seconds from the nozzle towards the anus which is the opening for defecation. Referring to Figs. 18A(a), 18B(b), 18C(c) and 23, it is also possible in this case to turn on the magnetic valve for defecation *a* only for discarding the cold rinsing liquid between the water heater 121 and the valve for 5 seconds into the toilet bowl 17, Fig. 18A(a), the magnet valve for defecation *b* then being turned off for 60 seconds with the aid of the 60-second timer of the magnetic valve *b* to discharge the rinsing liquid to the private parts, Fig. 18B(b), the valves *a* and *b* then being turned off, Fig. 18C(c). In this manner, there is no risk of directly discharging cold water towards the private parts. When the heat reserving means 125 such as an electric heater is provided between the water heater 121 and the valve, as shown in Figs. 20(A) and 20(B), the magnetic valve *c* for defecation may be activated for 60 seconds by the operation of the defecation timer-magnetic valve circuit 139 to turn on the valve *c*, Fig. 20(B), to discharge warm water to the private parts at 140.

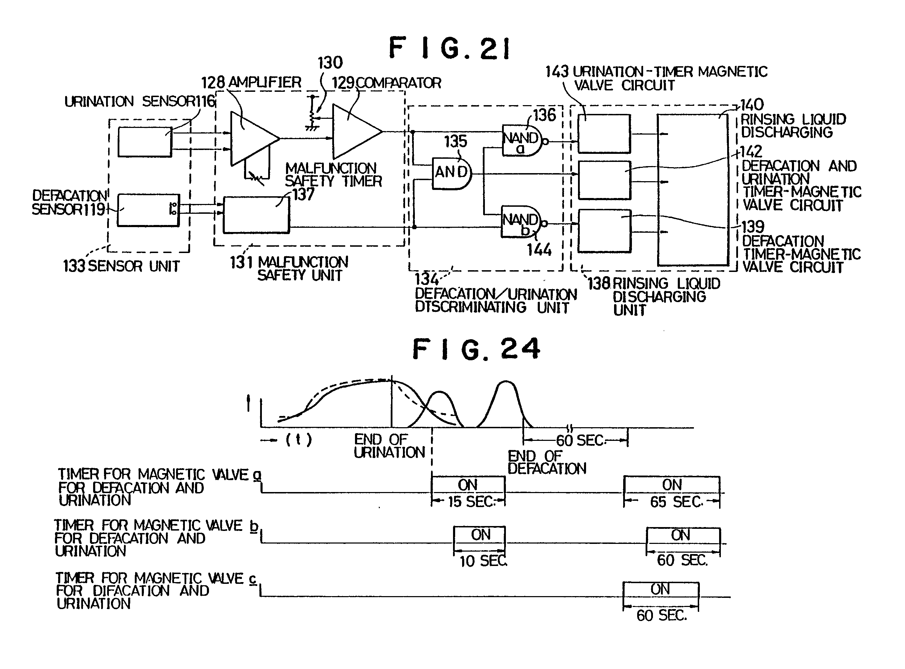

The signals from the pressure sensor 119 and the temperature or humidity sensor 116 of the sensor unit 133 indicating both defecation and urination, Fig. 24, are transmitted via malfunction safety unit 131 to ascertain excretion of both faeces and urine by the AND gate 135 of the discriminating unit 134. The output signal from the unit 134 indicating both defecation and urination is transmitted to the defecation/urination timer-magnetic valve 142 of the rinse liquid discharging unit 138. The defecation/urination timer-magnetic valve circuit 142 operates as follows: The signal from the urination sensor causes the magnetic valves *a* and *b* for urination between the water supply device and the water inlet port 123 to be turned on to permit the rinse liquid to be discharged for 10 seconds from the nozzle towards the guide pipe 114. The signal from the defecation sensor 119 causes magnetic valves for defecation *a* and *b* to be turned on to permit the rinsing liquid to be discharged at 140 from the nozzle towards the anus for 60 seconds after lapse of 60 seconds since the end of defecation. It will be noted that the magnetic valve *b* for urination or defecation is turned on 5 seconds before the valve *a* to drain cold water into the toilet bowl 17 instead of discharging it towards the private parts. Alternatively, when the heater or the like heat reserving means 25 is provided as described hereinabove, the timer-magnetic valve circuit 142 may be so designed that the timer of each magnetic valve *c* for defecation or urination is started in 60 seconds after the end of defecation to turn on the magnetic valves for urination and defecation *c, c* connecting to the nozzle 124 for defecation and to the nozzle 124' for urination, so as to discharge warm water at 140 towards the private parts.

**Claims**

1. A device for collecting urine and faeces excreted from a user's body and for automatically rinsing private parts of said user's body after the urination and/or defecation comprising a main body (1) including means for temporarily securing the user's body to the device while covering private parts such as anus and urethra, an inlet port (23) for introducing a rinsing liquid for rinsing the private parts, a drainage port (50) for discharging the used rinsing liquid with discharge materials out of the main body; and means (38) for releasing the rinsing liquid for a predetermined time towards the private parts such as anus and urethra,
said device being characterized by said main body (1) including means for sensing urination (16) and defecation (19), means (34) being provided for discriminating defecation and urination from each other on the basis of the output signals from said sensor means, and by said release of the rinsing liquids being carried out on the basis of the results of discrimination by said discriminating means.

2. A device according to claim 1 wherein said rinsing liquid releasing means including a resilient nozzle (24) provided to said inlet port and means (26) for deviating said resilient nozzle towards the one of the other of said private parts.

3. A device according to claim 1 wherein said rinsing liquid releasing means (38) includes at least two stationary nozzles (124, 124') provided to said inlet port and valves (a, b) operatively associated with these nozzles and wherein a selected one or ones of these valves is adapted to be opened on the basis of the results of discrimination by said discriminating means (38) for releasing the rinsing liquid from a water supply to the private parts through the thus opened valve or valves.

4. A device according to claim 1 wherein said temporary securing means includes a cushioning member (9, 10) provided to a body contacting portion (13, 6) of the main body of the device and profiled to water-tightly cover private parts of the female user, and means (8) for ensuring adherence of the device to the user's body.

5. A device according to claim 1 wherein said temporary securing means includes a washing pouch (48) for covering private parts of the male user and means (49) for attachment of the device to the user's body.

6. A device according to claim 1 wherein said inlet port (23) of the main body is connected to a water supply device (20) and said drain port (50) is connected to a toilet bowl.

7. A device according to claim 1 wherein the means for sensing the defecation and/or urination is selected from the group consisting of a temperature sensor, a light sensor, an odor sensor, a magnetic sensor and a pressure sensor.

8. A device according to claim 1 wherein said temporary securing means includes belts (3) and belt attachment rings (11).

9. A device according to claim 1 wherein a leakage safety cover (6) in the form of an elongated trough, is attached to said main body along with a lower base (7) and a spring (8), said cover being profiled to cover both the urethra and anus of the female body, said spring urging said cover into water-tight contact with the body portions about the urethra and anus.

10. A device according to claim 9 wherein a water-proofing cushioning member (10) is provided to the upper body-contacting portion of said leakage safety cover (6) and a shock-absorbing bellows-like member is annexed as a forward extension of the cushioning member.

11. A device according to claim 9 further comprising a deflector (15) provided in said leakage safety cover for refluxing the used rinsing liquid towards a toilet bowl.

12. A device according to claim 1 wherein said rinsing liquid is warm water and supplied from a water heater (21) connected to a water supply source (20).

13. A device according to claim 2 wherein said means (26) for deviating the resilient nozzle comprises iron segments (25-1, 25-2, 25-3) secured to said nozzle and electro-magnets (26-1,...,26-5) secured to the main body for selectively attracting the iron segments thereto.

14. A device according to claim 1 wherein the output signal, as sensed by said sensing means and indicating urination and/or defecation, is amplified and compared to a preset value, and the resulting signal in excess of a preset voltage actuates a timer (47) which transmits the rinsing liquid demand signal after lapse of a preset time since the end of urination and/or defecation.

15. A device according to claim 1 wherein the threshold sensing temperature of said sensing means (16, 19) is adjustable for compensation for seasonal changes in ambient atmospheric temperatures.

16. A device according to claim 1 wherein the output signal from said sensing means is passed through a malfunction safety unit (31).

17. A device according to claim 14 wherein said rinsing liquid demand signal is supplied to a defecation/urination discriminating unit (34).

18. A device according to claim 17 wherein a first nozzle-shifting electro-magnet-timer-magnet exciting circuit (45) of said discriminating unit causes a resilient nozzle to be directed towards the urethra of the user for a predetermined time.

19. A device according to claim 16 wherein the output signals from the malfunction safety unit (31) is supplied to the defecation/urination discriminating unit (34) to which the demand signal is also supplied.

20. A device according to claim 18 wherein a second nozzle-shifting electro-magnet-timer-magnet exciting circuit (44) of said discriminating unit causes a resilient nozzle to be directed towards the anus of the user for a predetermined time.

21. A device according to claim 20 wherein the output signals from said first and second exciting circuits of the discriminating unit are supplied to a rinsing liquid discharging unit (38).

22. A device according to claim 21 wherein a first timer and magnetic valve circuit (43) of the rinsing liquid discharging unit causes the rinsing liquid to be discharged for a first predetermined time.

23. A device according to claim 21 wherein a second timer and magnetic valve circuit (39) of the rinsing liquid discharging unit causes the rinsing liquid to be discharged for a second predetermined time.

24. A device according to claim 18 wherein said first nozzle-shifting electro-magnet-timer-magnet exciting circuit (45) of the discriminating unit (34) is so designed as to cause the nozzle to be directed to the user's urethra after a shorter time since the time of opening of the magnetic valve.

25. A device according to claim 20 wherein said second nozzle-shifting electro-magnet-timer-magnet exciting circuit (46) causes the nozzle to be directed towards the anus of the user after a shorter time since the time of opening of the valve.

26. A device according to claim 1 wherein signals, as sensed by said sensing means and indicating both defecation and urination, are supplied to a third nozzle-shifting electro-magnet for defecation/urination-timer-magnet exciting circuit (41) which, operates to sequentially excite the electro-magnets in such a manner that the urethra of the user's body is first rinsed for a first predetermined time by the rinsing liquid and the anus of the user's body is then rinsed for a second predetermined time by the rinsing liquid.

27. A device as claimed in claim 26 wherein the discharging of the rinsing liquid towards the urethra or anus is started a short time before nozzle shifting.

28. A device according to claim 1 wherein said temporary securing means includes a long tube (6) in which a urine guide tube (14) and a urination sensor (16) are accommodated and extended to close to a rinsing pouch (48) in which the opening of the urethra of the male user is accommodated.

29. A device according to claim 1 wherein the output signal from the said sensing means is passed through a malfunction safety unit (3), wherein the signal temporarily ceases to wait for end of defecation.

30. A device according to claim 1 wherein the output signal from said sensing means (16) and passed through a urination/defecation discriminating unit (34) and indicating urination is transmitted to a urination timer-magnetic valve circuit (43) of the rinse liquid discharging unit (38).

31. A device according to claim 1 wherein the output signal from said sensing means and passed through a urination/defecation discriminating unit and indicating both defecation and urination is transmitted to a defecation/urination timer-magnetic valve circuit (42) of the rinsing liquid discharging unit (38).

32. A device according to claim 1 wherein the output signal from said sensing means and passed through a urination/defecation discriminating unit and indicating defecation is transmitted to a defecation timer-magnetic valve circuit (39) of the rinsing liquid discharging unit (38).

33. A device according to claim 30 wherein said urination timer-magnetic valve circuit (43) is so designed that two magnetic valves are provided with one of the valves being opened some time before the opening of the other valve by the operation of the timer.

34. A device according to claim 31 wherein said defecation/urination timer-magnetic valve circuit (42) is so designed that two magnetic valves are provided with one of the valves being opened before the opening of the other valve by the operation of the timer.

35. A device according to claim 32 wherein said defecation timer-magnetic valve circuit (39) is so designed that two magnetic valves are provided with one of the valves being opened before the opening of the other valve by the operation of the timer.

## Patentansprüche

1. Vorrichtung zum Auffangen von Urin und Fäkalien nach Ausscheidung aus dem Körper eines Verwenders sowie zum automatischen Spülen der intimen Teile des Körpers eines Verwenders nach dem Urinieren und/oder Stühlgang, die die folgenden Teile umfaßt:
einen Hauptkörper (1) einschließlich Einrichtungen zum zeitweisen Befestigen des Körpers des Verwenders an der Vorrichtung, wobei Intimteile wie Anus und Harnröhre bedeckt werden,
eine Zulauföffnung (23) für die Zufuhr einer Spülflüssigkeit zum Spülen der Intimteile,
eine Ablauföffnung (50) für das Abführen der gebrauchten Spülflüssigkeit mit den Abfall-Materialien aus dem Hauptkörper und
Einrichtungen (38) zum Freisetzen der Spülflüssigkeit auf die Intimteile wie Anus und Harnröhre für eine vorbestimmte Zeitdauer,
wobei die Vorrichtung dadurch gekennzeichnet ist, daß der Hauptkörper (1) Einrichtungen zum Abtasten des Urinierens (16) und des Stuhlgangs (19) und Einrichtungen (34) umfaßt, die vorgesehen sind, um Stuhlgang un Urinieren voneinander auf der Basis der von der Abtast-Einrichtung abgegebenen Signale zu unterscheiden, und dadurch gekennzeichnet ist, daß die Freisetzung der Spülflüssigkeiten auf der Grundlage der Ergebnisse der Unterscheidung durch die Unterscheidungs-Einrichtung erfolgt.

2. Vorrichtung nach Anspruch 1, worin die Einrichtung zum Freisetzen der Spülflüssigkeit eine bewegliche Düse (24), die an der Einlaßöffnung vorgesehen ist und Einrichtungen (26) einschließt, um die bewegliche Düse auf das eine oder andere der Intimteile zu richten.

3. Vorrichtung nach Anspruch 1, worin die Einrichtung (38) zum Freisetzen der Spülflüssigkeit wenigstens zwei stationäre Düsen (124, 124') einschließt, die an der Einlaßöffnung vorgesehen sind, sowie Ventile (a, b), die in funktionsfähiger Weise mit diesen Düsen verbunden sind, und worin ein bestimmtes oder mehrere bestimmte Ventile aus dieser Gruppe auf der Grundlage der Ergebnisse der Unterscheidung durch die Unterscheidungs-Einrichtungen (38) geöffnet werden können, um Spülflüssigkeit aus einer Wasserversorgung durch das oder die geöffnete(n) Ventil(e) auf die Intimteile zu richten.

4. Vorrichtung nach Anspruch 1, worin die Einrichtung zum zweitweisen Befestigen ein Puffer-Teil (9, 10) einschließt, das an den mit dem Körper in Kontakt kommenden Teilen (13, 6) des Hauptkörpers der Vorrichtung vorgesehen ist und so gestaltet ist, daß es die Intimteile eines weiblichen Benutzers wasserdicht umschließt, und außerdem Einrichtungen (8) zur Sicherstellung der Befestigung der Vorrichtung am Körper des Benutzers.

5. Vorrichtung nach Anspruch 1, worin die Einrichtung zum zweitweisen Befestigen einen Waschbeutel (48) zum Abdecken der Intimteile eines männlichen Benutzers sowie Einrichtungen (49) zur Befestigung der Vorrichtung am Körper des Benutzers einschließt.

6. Vorrichtung nach Anspruch 1, worin die Einlaßöffnung (23) des Hauptkörpers mit einer Wasser-Versorgungsvorrichtung (20) und die Ablaßöffnung (50) mit einer Toilettenschüssel verbunden sind.

7. Vorrichtung nach Anspruch 1, worin die Einrichtung zum Abtasten des Stuhlgangs und/oder Urinierens ausgewählt ist aus der Gruppe Temperaturfühler, Lichtfühler, Geruchsfühler, magnetische Fühler und Druckfühler.

8. Vorrichtung nach Anspruch 1, worin die Einrichtung zum zeitweisen Refestigen Gurte (3) und Ringe (11) zur Befestigung der Gurte einschließt.

9. Vorrichtung nach Anspruch 1, worin eine Auslauf-sichere Abdeckung (6) in Form einer verlängerten Rinne an dem Hauptkörper angebracht ist, zusammen mit einer unteren Abstützung (7) und einer Feder (8), wobei die Abdeckung so gestaltet ist, daß sie sowohl die Harnröhre als auch den Anus des weiblichen Körpers bedecken, und die Feder die Abdeckung in wasserdichten Kontakt mit den Körperbereichen um die Harnröhre und den Anus herum bringt.

10. Vorrichtung nach Anspruch 9, worin ein Wasser-abdichtendes Pufferteil (10) am oberen, mit dem Körper in Kontakt kommenden Teil der Auslauf-sicheren Abdeckung (6) vorgesehen ist und ein Stoß-absorbierendes, balgartiges Teil als Verlängerung des Pufferteils nach vorne daran befestigt ist.

11. Vorrichtung nach Anspruch 9, wobei diese außerdem eine Ablenk-Platte (15) umfaßt, die in der Auslauf-sicheren Abdeckung vorgesehen ist, um die gebrauchte Spülflüssigkeit einer Toilettenschüssel zuzuleiten.

12. Vorrichtung nach Anspruch 1, worin die Spülflüssigkeit warmes Wasser ist und aus einem Wasser-Heizer (21) zugeleitet wird, der mit einer Zuleitung (20) für die Wasserversorgung verbunden ist.

13. Vorrichtung nach Anspruch 2, worin die Einrichtung (26) zum Dirigieren der beweglichen Düse Eisen-Segmente (25-1, 25-2, 25-3) umfaßt, die an der Düse befestigt sind, sowie Elektromagneten (26-1, 26-2, 26-3), die an dem Hauptkörper befestigt sind, um selektiv die Eisen-Segmente anzuziehen.

14. Vorrichtung nach Anspruch 1, worin das Ausgangs-Signal so, wie es durch die Abtast-Einrichtung abgenommen wurde und Urinieren und/oder Stuhlgang anzeigt, vervielfältigt und mit einem voreingestellten Wert verglichen wird, und das resultierende Signal, soweit es eine voreingestellte Spannung übersteigt, einen Zeitgeber (47) in Gang setzt, der das Signal für den Bedarf von Spülflüssigkeit nach Ablauf einer voreingestellten Zeit nach dem Ende des Urinierens und/oder Stuhlgangs übermittelt.

15. Vorrichtung nach Anspruch 1, worin die Schwellen-Abtasttemperatur der Abtast-Einrichtung (16, 19) zur Kompensation saisonaler Änderungen der Umgebungstemperaturen einstellbar ist.

16. Vorrichtung nach Anspruch 1, worin das Ausgangs-Signal aus der Abtast-Einrichtung über eine Störungs-Sicherungseinheit (31) geleitet wird.

17. Vorrichtung nach Anspruch 14, worin das Signal für den Bedarf von Spülflüssigkeit zu einer Einheit (34) zur Unterscheidung von Stuhlgang und Urinierten geleitet wird.

18. Vorrichtung nach Anspruch 17, worin ein erster, den Zeitgebermagneten elektromagnetisch anregender Kreislauf (45) der Unterscheidungseinheit zur Verstellung der Düsen das Einstellen einer beweglichen Düse auf die Harnröhre des Benutzers für eine vorbestimmte Zeit bewirkt.

19. Vorrichtung nach Anspruch 16, worin die Ausgangssignale der Störungs-Sicherheitseinheit (31) zur Einheit (34) zur Unterscheidung von Stuhlgang/Urinieren geleitet werden, zu der auch das Anforderungs-Signal geleitet wird.

20. Vorrichtung nach Anspruch 18, worin ein zweiter, den Zeitgeber-Magneten elektromagnetisch anregender Kreislauf (44) der Unterscheidungs-Einheit zur Verstellung der Düse die Einsetellung einer beweglichen Düse auf den Anus des Benutzers für eine vorbestimmte Zeit bewirkt.

21. Vorrichtung nach Anspruch 20, worin die Ausgangs-Signale der ersten und zweiten Anregungs-Kreisläufe der Unterscheidungs-Einheit zu einer Einheit (38) zum Abführen der Spülflüssigkeit geleitet werden.

22. Vorrichtung nach Anspruch 21, worin ein erster, eine Zeitschaltuhr und ein magnetisches Ventil enthaltender Kreislauf (43) der Einheit zum Abführen der Spülflüssigkeit das Abführen der Spülflüssigkeit für eine erste vorbestimmte Zeit bewirkt.

23. Vorrichtung nach Anspruch 21, worin ein zweiter, eine Zeitschaltuhr und ein magnetisches Ventil enthaltender Kreislauf (39) der Einheit zur Abführung der Spülflüssigkeit das Abführen der Spüflüssigkeit für eine zweite vorbestimmte Zeit bewirkt.

24. Vorrichtung nach Anspruch 18, worin der erste, den Magneten der Zeitschaltuhr elektromagnetisch anregende Kreislauf (45) der Unterscheidungs-Einheit (34) zur Verstellung der Düsen so gestaltet ist, daß er die Ausrichtung der Düse auf die Harnröhre des Benutzers nach einer kürzeren Zeit bewirkt als die Zeitdauer der Öffnung des magnetischen Ventils.

25. Vorrichtung nach Anspruch 20, worin der zweite, den Zeitschalter-Magneten elektromagnetisch anregende Kreislauf (46) zur Verstellung der Düsen eine Ausrichtung der Düse auf den Anus des Benutzers nach einer kürzeren Zeitdauer bewirkt als die Zeitdauer der Öffnung des Ventils.

26. Vorrichtung nach Anspruch 1, worin die Signale, soweit sie durch die Abtast-Einrichtungen abgetastet werden und sowohl Stuhlgang als auch Urinieren anzeigen, einem dritten Düsenverstellenden Elektromagnet für einen den Magneten des Stuhlgangs/Urinierungs-Zeitschalters anregenden Kreislauf (41) zugeführt werden, der in der Weise arbeitet, daß er aufeinanderfolgend die Elektromagneten in einer Weise anregt, daß zuerst die Harnröhre des Körpers des Benutzers für eine erste vorbestimmte Zeit mit Spülflüssigkeit gespült und danach der Anus des Körpers des Benutzers für eine zweite vorbestimmte Zeit mit Spülflüssigkeit gespült wird.

27. Vorrichtung nach Anspruch 26, worin das Abführen der gegen die Harnröhre oder den Anus gerichteten Spülflüssigkeit eine kurze Zeit vor der Verstellung der Düsen gestartet wird.

28. Vorrichtung nach Anspruch 1, worin die Einrichtung zum zeitweisen Befestigen ein langes Rohr (6) einschließt, in dem ein Urin-Leitungsrohr (14) und ein Urinierungs-Sensor (16) untergebracht und in der Weise verlängert sind, daß sie einen Spül-Beutel (48) verschließen, in dem die Öffnung der Harnröhre eines männlichen Benutzers untergebracht ist.

29. Vorrichtung nach Anspruch 1, worin das Ausgangs-Signal aus der Abtast-Einrichtung über eine Störungs-Sicherheitseinheit (3) geleitet wird, worin das Signal vorübergehend unterbrochen wird, um das Ende des Stuhlgangs abzuwarten.

30. Vorrichtung nach Anspruch 1, worin das Ausgangs-Signal aus der Abtast-Vorrichtung (16), das zu der Unterscheidungseinheit (34) zwischen Urinieren und Stuhlgang geleitet wurde und Urinieren anzeigt, zu einem Urinierungs-Zeitschalter-Magnetventil-Kreislauf (43) der Einheit (38) zur Abführung der Spülflüssigkeit übertragen wird.

31. Vorrichtung nach Anspruch 1, worin das Ausgangs-Signal aus der Abtast-Einrichtung, das zu der Unterscheidungs-Einheit zwischen Urinieren und Stuhlgang geleitet wurde und sowohl Stuhlgang als ach Urinieren anzeigt, zu einem Stuhlgang/Urinieren - Zeitschalter - Magnetventil-Kreislauf (42) der Einheit (39) zum Abführen der Spülflüssigkeit geleitet wird.

32. Vorrichtung nach Anspruch 1, worin das Ausgangs-Signal aus der Abtast-Einrichtung, das zu der Unterscheidungs-Einheit zwischen Urinieren und Stuhlgang geleitet wird und Stuhlgang anzeigt, zu einem Stuhlgang-Zeitschalter-Magnetventil-Kreislauf (39) der Einheit (38) zur Abführung der Spülflüssigkeit geleitet wird.

33. Vorrichtung nach Anspruch 30, worin der Urinierungs - Zeitschalter - Magnetventil - Kreislauf (43) so gestaltet ist, daß zwei Magnetventile vorgesehen werden, wobei eines der Ventile einige Zeit vor dem Öffnen des anderen Ventils aufgrund der Tätigkeit des Zeitschalters geöffnet wird.

34. Vorrichtung nach Anspruch 31, worin der Stuhlgang/Urinierungs-Zeitschalter - Magnetventil-Kreislauf (42) so gestaltet ist, daß zwei Magnetventile vorgesehen werden, wobei eines der Ventile vor dem Öffnen des anderen Ventils durch die Tätigkeit des Zeitschalters geöffnet wird.

35. Vorrichtung nach Anspruch 32, worin der Stuhlgang-Zeitschalter - Magnetventil - Kreislauf (39) so gestaltet ist, daß zwei Magnetventile vorgesehen werden, wobei eines der Ventile vor dem Öffnen des anderen Ventils durch die Tätigkeit des Zeitschalters geöffnet wird.

## Revendications

1. Dispositif pour recueillir l'urine et les matières fécales expulsées du corps d'un utilisateur et pour rincer automatiquement les parties intimes dudit corps de l'utilisateur après expulsion de l'urine et/ou défécation, comportant un corps principal (1) comprenant des moyens pour fixer temporairement le corps de l'utilisateur au dispositif en en couvrant les parties intimes telles que l'anus et l'urètre, un orifice d'entrée (23) pour introduire un liquide de rinçage pour rincer les parties intimes, un orifice d'évacuation (50) pour évacuer, hors du corps principal, le liquide de rinçage utilisé ainsi que les matières à évacuer; et comportant aussi des moyens (38) pour laisser échapper le liquide de rinçage pendant une période de temps prédéterminée en direction des parties intimes telles que l'anus et l'urètre,

ledit dispositif étant caractérisé par le fait que ledit corps principal (1) comprend des moyens pour détecter l'expulsion de l'urine (16) et la défécation (19), des moyens (34) étant prévus pour distinguer l'une de l'autre la défécation et l'expulsion de l'urine sur la base des signaux de sortie provenant desdits moyens de détection, et par le fait que ledit échappement du liquide de rinçage s'effectue sur la base des résultats de la distinction opérée par lesdits moyens de distinction.

2. Dispositif selon la revendication 1, dans lequel lesdits moyens permetttant de laisser échapper le liquide de rinçage comprennent une buse souple (24) montée sur ledit orifice d'entrée et des moyens (26) pour dévier ladite buse souple en direction de l'une ou de l'autre desdites parties intimes.

3. Dispositif selon la revendication 1, dans lequel lesdits moyens (38) permettant de laisser échapper du liquide de rinçage comprennent au moins deux buses fixes (124, 124') montées sur ledit orifice d'entrée et des vannes (a, b) opérationnellement associées à ces buses et dans lequel une ou plusieurs, sélectionnées de ces vannes sont conçues pour s'ouvrir sur la base des résultats de la distinction opérée par les moyens de distinction (38), pour laisser échapper le liquide de rinçage provenant de l'arrivée d'eau vers les parties intimes, en passant par la vanne ou les vannes ainsi ouvertes.

4. Dispositif selon la revendication 1, dans lequel lesdits moyens de fixation temporaire comprennent un élément rembourré (9, 10) monté sur un portion (13, 6) du corps principal du dispositif qui vient au contact du corps et profilé de façon à recouvrir, avec étanchéité à l'eau, les parties intimes d'un utilisateur femme, ainsi que des moyens (8) pour assurer l'adhérence du dispositif au corps de l'utilisateur.

5. Dispositif selon la revendication 1, dans lequel lesdits moyens de fixation temporaire comprennent une poche de lavage (48) pour couvrir les parties intimes de l'utilisateur mâle et des moyens (49) pour fixer le dispositif au corps de l'utilisateur.

6. Dispositif selon la revendication 1, dans lequel ledit orifice d'entrée (23) du corps principal est relié à un dispositif (20) d'alimentation en eau et ledit orifice du vidange (50) est relié à une cuvette de toilette.

7. Dispositif selon la revendication 1, dans lequel les moyens de détecter la défécation et/ou l'expulsion d'urine sont choisis dans le groupe constitué d'un détecteur de température, d'un détecteur de lumière, d'un détecteur d'odeur, d'un détecteur magnétique et d'un détecteur de pression.

8. Dispositif selon la revendication 1, dans lequel lesdits moyens de fixation temporaire comprennent des ceintures (3) et des anneaux (11) de fixation des ceintures.

9. Dispositif selon la revendication 1, dans lequel un couvercle (6) de sécurité en cas de fuite, sous forme d'une goulotte de forme allongée, est fixé audit corps principal avec une embase inférieure (7) et un ressort (8), ledit couvercle étant profilé pour couvrir à la fois l'urètre et l'anus du corps de la femme, ledit ressort contraignant ledit couvercle à venir en contact étanche à l'eau avec les portions du corps situées autour de l'urètre et de l'anus.

10. Dispositif selon la revendication 9, dans lequel un élément rembourré (10) d'étanchéité à l'eau est prévu sur la portion supérieure, venant en contact avec le corps, dudit couvercle (6) de sécurité en cas de fuite, et dans lequel un élément du type soufflet, absorbant les chocs, est joint en prolongement avant de l'élément rembourré.

11. Dispositif selon la revendication 9, comportant en outre un déflecteur (15) prévu dans ledit couvercle de sécurité en cas de fuite pour renvoyer en direction d'une cuvette de toilette le liquide de rinçage utilisé.

12. Dispositif selon la revendication 1, dans lequel ledit liquide de rinçage est de l'eau chaude provenant d'un chauffe-eau (21) relié à une source (20) d'alimentation en eau.

13. Dispositif selon la revendication 2, dans lequel lesdits moyens (26) prévus pour dévier la buse souple comportent des segments de fer deux (25-1, 25-2, 25-3) fixés à ladite buse et des électro-aimants (26-1..., 26-5) fixés au corps principal pour attirer sélectivement vers eux les segments de fer deux.

14. Dispositif selon la revendication 1, dans lequel le signal de sortie, tel que détecté par lesdits moyens de détection et indiquant l'expulsion d'urine et/ou la défécation, est amplifié et comparé à une valeur prédéterminée, et dans lequel le signal résultant, s'il dépasse une valeur prédéterminée de la tension, actionne un temporisateur (47) qui transmet le signal de demande de liquide de rinçage après écoulement d'une durée prédéterminée à partir de la fin de la défécation et/ou de l'expulsion d'urine.

15. Dispositif selon la revendication 1, dans lequel le seuil de détection de température desdits moyens de détection (16, 19) peut se régler pour compenser les changements saisonniers de la température atmosphérique ambiante.

16. Dispositif selon la revendication 1, dans lequel le signal de sortie provenant desdits moyens de détection passe par une unité de sécurité en cas de fonctionnement erroné (31).

17. Dispositif selon la revendication 14 dans lequel le signal de demande de liquide de rinçage est envoyé à une unité (34) de distinction défécation/expulsion d'urine.

18. Dispositif selon la revendication 17 dans lequel un premier circuit (45) d'excitation de l'électro-aimant temporisé de décalage de la buse de ladite unité de distinction fait qu'une buse souple sera dirigée en direction de l'urètre de l'utilisateur pendant un temps prédéterminé.

19. Dispositif selon la revendication 16, dans lequel les signaux de sortie provenant de l'unité de sécurité en cas de fonctionnement erroné (31) sont envoyés à l'unité (34) de distinction défécation/expulsion d'urine à laquelle le signal de demande est également envoyé.

20. Dispositif selon la revendication 18 dans lequel un second circuit (44) d'excitation des électro-aimants temporisés du décalage de la buse de ladite unité de distinction fait qu'une buse souple sera dirigée vers l'anus de l'utilisateur pendant une période prédéterminée.

21. Dispositif selon la revendication 20, dans lequel les signaux de sortie provenant dudit premier et du second circuits d'excitation de l'unité de distinction sont envoyés à une unité (38) d'envoi de liquide de rinçage.

22. Dispositif selon la revendication 21, dans lequel un premier circuit (43) de temporisateur et d'électrovanne de l'unité d'envoi du liquide de rinçage fait que le liquide de rinçage sera envoyé pendant une première période prédéterminée.

23. Dispositif selon la revendication 21, dans lequel un second circuit (39) de temporisateur et d'électrovanne de l'unité d'envoi du liquide de rinçage fait que le liquide de rinçage sera envoyé pendant une seconde période prédéterminée.

24. Dispositif selon la revendication 18 dans lequel ledit premier circuit (45) d'excitation des électro-aimants temporisés de décalage de la buse de l'unité de distinction 34 est conçu de façon à faire que la buse soit dirigée vers l'urètre de l'utilisateur un court délai après l'instant d'ouverture de l'électrovanne.

25. Dispositif selon la revendication 20, dans lequel le second circuit (46) d'excitation des électro-aimants temporisés de décalage de la buse fait que la buse sera dirigée vers l'anus de l'utilisateur un court délai après l'instant d'ouverture de la vanne.

26. Dispositif selon la revendication 1, dans lequel des signaux, tels que détectés par lesdits moyens de détection et indiquant à la fois la défécation et l'expulsion d'urine, sont envoyés à un troisième circuit (41) d'excitation des électro-aimants temporisés de décalage de la buse pour le cas de défécation/expulsion d'urine, circuit qui opère pour exciter séquentiellement les électro-aimants de façon que l'urètre du corps de l'utilisateur soit tout d'abord rincé pendant une période de temps prédéterminée par le liquide de rinçage puis que l'anus du corps de l'utilisateur soit ensuite rincé pendant une seconde période de temps prédéterminée par le liquide de rinçage.

27. Dispositif selon la revendication 26, dans lequel l'envoi du liquide de rinçage en direction de l'urètre ou de l'anus démarre un court délai après le décalage de la buse.

28. Dispositif selon la revendication 1, dans lequel lesdits moyens de fixation temporaire comprennent un long tube (6) dans lequel un tube (8) de guidage de l'urine et un détecteur (16) de l'expulsion d'urine sont logés et s'étendent très près d'une poche de rinçage (48) dans laquelle se loge l'ouverture de l'urètre de l'utilisateur mâle.

29. Dispositif selon la revendication 1, dans lequel le signal de sortie provenant desdits moyens de détection passe par une unité de sécurité en cas de fonctionnement erroné (37), et dans lequel le signal cesse temporairement pour attendre la fin de la défécation.

30. Dispositif selon la revendication 1, dans lequel le signal de sortie qui provient desdits moyens de détection (16) et qui est passé par l'unité (34) de distinction expulsion d'urine/défécation, et qui indique l'expulsion d'urine, est envoyé à un circuit (43) temporisateur-électrovanne, pour le cas d'expulsion d'urine, de l'unité (38) d'envoi du liquide de rinçage.

31. Dispositif selon la revendication 1, dans lequel le signal de srotie qui provient desdits moyens de détection et qui est passé par l'unité de distinction expulsion d'urine/défécation et qui indique à la fois la défécation et l'expulsion d'urine est envoyé à un circuit (42) temporisateur-électrovanne, pour le cas de défécation/expulsion d'urine, de l'unité (38) d'envoi du liquide de rinçage.

32. Dispositif selon la revendication 1, dans lequel le signal de sortie qui provient desdits moyens de détection et qui est passé par l'unité de distinction expulsion d'urine/défécation et qui indique la défécation est envoyé à un circuit (39) temporisateur-électrovanne, pour le cas de défécation, de l'unité (38) d'envoi du liquide de rinçage.

33. Dispositif selon la revendication 30, dans lequel ledit circuit (43) temporisateur-électrovanne pour le cas d'expulsion d'urine est conçu de façon que deux électrovannes soient prévues, l'une des vannes étant ouverte un certain temps avant l'ouverture de l'autre vanne par la manoeuvre du temporisateur.

34. Dispositif selon la revendication 31 dans lequel ledit circuit (42) temporisateur-électrovanne, pour le cas de défécation/expulsion d'urine, est conçu de façon que deux électrovannes soient prévues, d'une des vannes étant ouverte avant l'ouverture de l'autre vanne par manoeuvre du temporisateur.

35. Dispositif selon la revendication 32 dans lequel ledit circuit (39) temporisateur-électrovanne, en cas de défécation, est conçu de façon que deux électrovannes soient prévues, l'une des vannes étant ouverte avant l'ouverture de l'autre vanne par la manoeuvre du temporisateur.

FIG. I

FIG. 2

FIG. 3

FIG. 4

EP 0 200 174 B1

FIG.7

FIG.6

FIG.5

FIG.8

FIG.9

FIG.11

FIG.12

EP 0 200 174 B1

EP 0 200 174 B1

FIG.10

FIG.13

4

EP 0 200 174 B1

# FIG. 14

NOZZLE-SHIFTING ELECTRO-MAGNET EXCITING CIRCUIT FOR DEFACATION AND URINATION

45 NOZZLE-SHIFTING ELECTRO-MAGNET EXCITING CIRCUIT FOR URINATION

40 RINSING LIQUID DISCHARGING

42 DEFACATION AND URINATION TIMER-MAGNETIC VALVE CIRCUIT

39 DEFACATION TIMER-MAGNETIC VALVE CIRCUIT

43 URINATION TIMER-MAGNETIC VALVE CIRCUIT

38 RINSING LIQUID DISCHARGING UNIT

NOZZLE-SHIFTING ELECTRO-MAGNET EXCITING CIRCUIT FOR DEFACATION AND URINATION 41

36 NAND a

44 NAND b

46 NOZZLE-SHIFTING ELECTRO-HAGNET EXCITING CIRCUIT FOR DEFACATION

35 AND

47 TIMER

30

28 AMPLIFIER

32

29 COMPARATOR

37 MALFUNCTION SAFETY TIMER

31 MALFUNCTION SAFETY UNIT

34 DEFACATION/URINATION DISCRIMINATING UNIT

16 URINATION SENSOR

DEFACATION SENSOR 19

33 SENSOR UNIT

# FIG. 15

(PRESSURE)

PRESSURE SENSOR

5 ᵐ/m

(TIME)

END OF DEFACATION

NOZZLE-SHIFTING ELECTRO-MAGNET EXCITING CIRCUIT FOR DEFACATION

ON 60 SEC.

60 SEC.

DEFACATION TIMER-MAGNETIC VALVE CIRCUIT

ON 60 SEC.

# FIG. 16

— TEMP SENSOR

---- HUMIDITY SENSOR

(TIME)

END OF URINATION

90 SEC.

NOZZLE-SHIFTING ELECTRO-MAGNET EXCITING CIRCUIT FOR URINATION

ON 20 SEC.

URINATION TIMER-MAGNETIC VALVE CIRCUIT

ON 25 SEC.

5

# FIG.17

PRESSURE (VOLTAGE)

5 m/m

90 SEC.

END OF URINATION

60 SEC.

END OF DEFACATION

NOZZLE-SHIFTING ELECTRO-MAGNET EXCITING CIRCUIT FOR DEFACATION AND URINATION

ON | 20 SEC.

ON | 60 SEC.

DEFACATION AND URINATION TIMER-MAGNETIC VALVE CIRCUIT

ON | 25 SEC.

ON | 65 SEC.

# FIG.19

a b
b a
22
21

# FIG.20A

TWO-WAY VALVE FOR URINATION ON

c URINATION NOZZLE VALVE ON
c
22 25

# FIG.20B

c DEFACATION NOZZLE VALVE ON
c
25 22

TWO-WAY VALVE FOR DEFACATION ON

EP 0 200 174 B1

# FIG.18A

COLD WATER DISCARDING

(i)

22

a b OFF

TWO-WAY VALVE FOR URINATION ON a

b COLD WATER DISCARDING

(a) 22

a b

OFF

TWO-WAY VALVE FOR DEFACATION ON a

b COLD WATER DISCARDING

# FIG.18B

RINSING

(ii)

a b URINATION NOZZLE VALVE ON

WARM WATER

22 a

b

(b) 22

a b

b WARM WATER

TWO-WAY VALVE FOR DEFACATION ON a

DEFACATION NOZZLE VALVE ON

# FIG.18C

END OF RINSING

(iii)

a b

22

a b

(c)

a b

22

a b

# FIG.22

—— TEMP SENSOR (V)
---- HUMIDITY SENSOR

→(t)     END OF URINATION

TIMER FOR MAGNETIC VALVE a FOR URINATION

ON
— 15 SEC.

TIMER FOR MAGNETIC VALVE b FOR URINATION

ON
10 SEC.

TIMER FOR MAGNETIC VALVE c FOR URINATION

ON
10 SEC.

# FIG.23

PRESSURE SENSOR (V)

→(t)     END OF DEFACATION     60 SEC.

TIMER FOR MAGNETIC VALVE a FOR DEFACATION

ON
— 65 SEC.

TIMER FOR MAGNETIC VALVE b FOR DEFACATION

ON
60 SEC.

TIMER FOR MAGNETIC VALVE c FOR DEFACATION

ON
60 SEC.

# F I G. 21

URINATION SENSOR 116

128 AMPLIFIER

130

129 COMPARATOR

143 URINATION-TIMER MAGNETIC VALVE CIRCUIT

136

135

AND

NAND a

140 RINSING LIQUID DISCHARGING

MALFUNCTION SAFETY TIMER

137

142 DEFACATION AND URINATION TIMER-MAGNETIC VALVE CIRCUIT

DEFACATION SENSOR 119

NAND b

144

139 DEFACATION TIMER-MAGNETIC VALVE CIRCUIT

133 SENSOR UNIT

131 MALFUNCTION SAFETY UNIT

134 DEFACATION/URINATION DTSCRIMINATING UNIT

138 RINSING LIQUID DISCHARGING UNIT

# F I G. 24

$I$

— (t)

END OF URINATION

60 SEC.

END OF DEFACATION

TIMER FOR MAGNETIC VALVE a FOR DEFACATION AND URINATION

ON

15 SEC.

ON

65 SEC.

TIMER FOR MAGNETIC VALVE b FOR DEFACATION AND URINATION

ON

10 SEC.

ON

60 SEC.

TIMER FOR MAGNETIC VALVE c FOR DIFACATION AND URINATION

ON

60 SEC.

EP 0 200 174 B1